# EUROPEAN PATENT APPLICATION

(11) **EP 3 754 283 A1**
(43) Date of publication of application: **23.12.2020**
(21) Application number: 19775429.4
(22) Date of filing: 18.03.2019
(51) Int. Cl.: F28F 19/00, A01N 25/00, A01N 25/28, A01P 3/00, A61L 2/18, F24F 13/22, F28F 13/18

(54) **DRUG-CONTAINING CAPSULE, AND COMPONENT FOR AIR TREATMENT DEVICE**

(30) Priority: 29.03.2018 JP 2018065107
(71) Applicant: DAIKIN INDUSTRIES, LTD., Osaka 530-8323 (JP)
(72) Inventor: DONOSHITA Yuka, Osaka-shi, Osaka 530-8323 (JP); SAKAMOTO Masako, Osaka-shi, Osaka 530-8323 (JP)
(74) Representative: Hoffmann Eitle
(86) International application number: PCT/JP2019/011146
(87) International publication number: WO 2019/188489

(57) **Abstract**

A drug-containing capsule (20, 30, 40) includes a capsule material (21, 31, 41) and a drug (22, 32, 42) disposed within the capsule material (21, 31, 41) and having a sterilization action for a specific microorganism. The capsule material (21, 31, 41) includes a degradable part (21a, 31a, 41a) formed of a raw material that is caused to biodegrade by the specific microorganism. This results in suppression of release of the drug while the specific microorganism does not proliferate.

## Description

### Technical Field

The present disclosure relates to a drug-containing capsule and a component for an air treatment device.

### Background Art

There is a known fin including a base member and a hydrophilic film and used for a heat exchanger (for example, Patent Literature 1). The hydrophilic film includes a plurality of types of drug particles that have a surface covered with a capsule material having controlled dissolubility in water and that are different from each other in the timing of exerting the drug action. In this way, the drug particles can exert the drug action for a long term.

### Citation List

### Patent Literature

PTL 1: Japanese Unexamined Patent Application Publication No. 2016-125698

### Summary of Invention

### Technical Problem

However, the above-described capsule material has controlled dissolubility in water and hence the capsule material having been in contact with water for a certain period of time can release the drug particles disposed therein. Specifically, in the case of generation of water near the capsule material, even when a specific microorganism that is the target of the drug action does not proliferate, the drug particles disposed therein are released, so that the drug particles may be wasted without effectively exerting the drug action.

An object of the present disclosure is to suppress release of the drug while the specific microorganism does not proliferate.

### Solution to Problem

A first embodiment of the present disclosure is directed to a drug-containing capsule (20, 30, 40) including a capsule material (21, 31, 41) and a drug (22, 32, 42) disposed within the capsule material (21, 31, 41) and having a sterilization action for a specific microorganism. The capsule material (21, 31, 41) includes a degradable part (21a, 31a, 41a) formed of a raw material that is caused to biodegrade by the specific microorganism.

In the first embodiment, in the capsule material (21, 31, 41), the specific microorganism causes biodegradation of the degradable part (21a, 31a, 41a) at the spot of proliferation of the microorganism. This results in release of the drug (22, 32, 42) from within the capsule material (21, 31, 41) to destroy the proliferating microorganism. Thus, the drug (22, 32, 42) is not released until the specific microorganism has proliferated to some extent.

In a second embodiment of the present disclosure, in the first embodiment, the specific microorganism is a mold, a yeast, or a bacterium proliferating in an air treatment device (1).

The second embodiment provides a drug-containing capsule (20, 30, 40) that effectively acts against the mold, the yeast, or the bacterium proliferating in the air treatment device (1).

In a third embodiment of the present disclosure, in the second embodiment, the mold includes at least one of Penicillium molds, Cladosporium molds, Aspergillus molds, Toxicocladosporium molds, Engyodontium molds, Aureobasidium molds, Alternaria molds, Paecilomyces molds, Tricoderma molds, Nigrospora molds, Chaetomium molds, Ulocladium molds, Fusarium molds, Eurotium molds, Curvularia molds, Rhodotorula molds, Chaetomium molds, Mucor molds, and Rhizopus molds; the yeast includes at least one of true fungi that are collectively called yeast; and the bacterium includes at least one of Arthrobacter bacteria, Baccilus bacteria, Serratia bacteria, and Staphylococcus aureus.

The third embodiment provides a drug-containing capsule (20, 30, 40) that effectively acts against the specific mold, yeast, or bacterium.

In a fourth embodiment of the present disclosure, in any one of the first to third embodiments, the raw material that is caused to biodegrade by the specific microorganism is a polymer including caprolactone or a polymer including ethylene adipate.

The fourth embodiment provides a drug-containing capsule (20, 30, 40) that effectively acts against a microorganism that causes biodegradation of such a polymer.

In a fifth embodiment of the present disclosure, in any one of the first to fourth embodiments, the capsule material (21) includes a plurality of the capsule materials (21) disposed as layers, and the drug (22) is disposed within each of the capsule materials (21).

In the fifth embodiment, upon biodegradation of the outermost capsule material (21), the drug (22) disposed therein is released to destroy the specific microorganism. Subsequently, upon biodegradation of the inner capsule material (21), the drug (22) disposed therein is released to destroy the specific microorganism. In this way, such drug-containing capsules (20) each provide a drug action a plurality of times.

A sixth embodiment of the present disclosure is directed to a component (10) for an air treatment device (1). This component (10) includes a film (13) including the drug-containing capsule (20, 30, 40) according to any one of the first to fifth embodiments.

In the sixth embodiment, in the air treatment device (1), proliferation of the specific microorganism on the component (10) including the film (13) including the drug-containing capsule (20, 30, 40) is effectively suppressed.

In a seventh embodiment of the present disclosure, in the sixth embodiment, the film (13) includes a plurality of types of the drug-containing capsules (20, 30, 40), and the plurality of types of the drug-containing capsules (20, 30, 40) are different from each other in a thickness (T1, T2, T3) of the degradable part (21a, 31a, 41a) of the capsule material (21, 31, 41).

In the seventh embodiment, the capsule materials (21, 31, 41) include the degradable parts (21a, 31a, 41a) that have thicknesses (T1, T2, T3) different from each other, and perforation due to biodegradation occurs at different timings, so that the drugs (22, 32, 42) disposed therein are released at timings different from each other. Therefore, the plurality of types of the drug-containing capsules (20, 30, 40) as a whole exert the drug action for a longer term.

An eighth embodiment of the present disclosure is directed to a drug-containing capsule (20, 30, 40) used for an air treatment device (1). The drug-containing capsule (20, 30, 40) includes a capsule material (21, 31, 41) including a degradable part (21a, 31a, 41a) formed of a polymer including caprolactone or a polymer including ethylene adipate, and a drug (22, 32, 42) disposed within the capsule material (21, 31, 41) and having a sterilization action for a microorganism.

In the eighth embodiment, the degradable part (21a, 31a, 41a) of the capsule material (21, 31, 41) is caused to biodegrade by a microorganism proliferating in the air treatment device (1). This causes release of the drug (22, 32, 42) from within the capsule material (21, 31, 41) to destroy the microorganism. Therefore, in the air treatment device (1), the drug action of the drug (22, 32, 42) is exerted at the spot and timing of proliferation of the microorganism.

### Brief Description of Drawings

[Fig. 1] Fig. 1 is a schematic view illustrating the configuration of an air conditioning device according to an embodiment.
[Fig. 2] Fig. 2 is a sectional view schematically illustrating a near-surface region of a heat exchanger of an air conditioning device according to an embodiment.
[Fig. 3] Fig. 3 illustrates an example of a drug-containing capsule according to an embodiment.
[Fig. 4] Fig. 4 illustrates an example of a drug-containing capsule according to an embodiment.
[Fig. 5] Fig. 5 illustrates an example of a drug-containing capsule according to an embodiment.
[Fig. 6] Fig. 6 illustrates an example of a drug-containing capsule according to another embodiment.
[Fig. 7] Fig. 7 illustrates an example of a drug-containing capsule according to another embodiment. Description of Embodiments

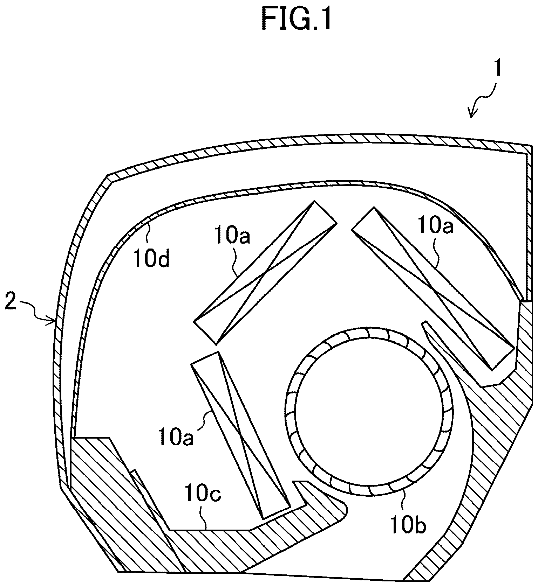

A drug-containing capsule (20, 30, 40) according to an embodiment is used for an air treatment device (1). As illustrated in Fig. 1, the air treatment device (1) according to the embodiment is constituted by an air conditioning device (1). This air conditioning device (1) includes a casing (2), a heat exchanger (10a), a fan (10b), a drain pan (10c), a filter (10d), and a flap (not shown) that are each contained in the casing (2), and a duct (not shown). These individually constitute components (10).

### Configurations of near-surface regions of components

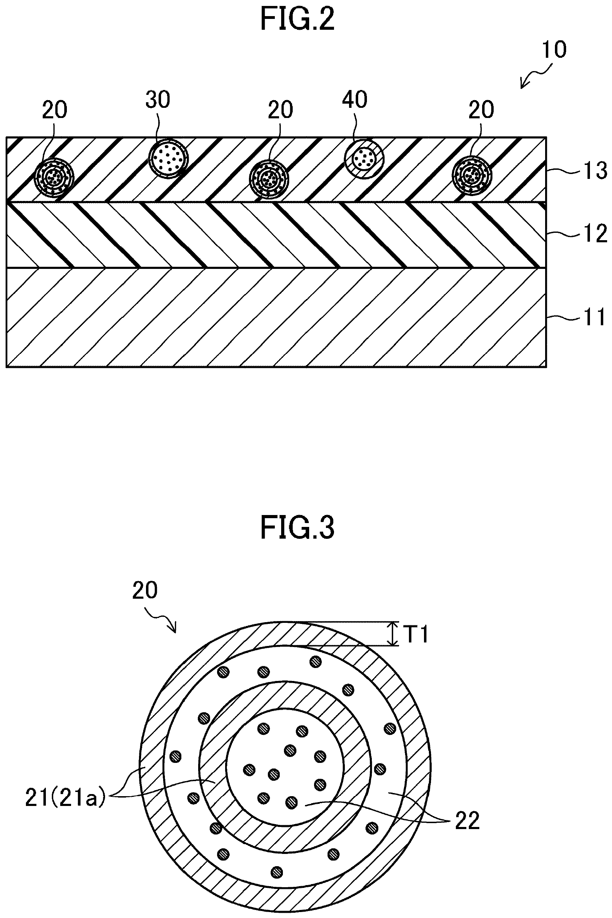

As illustrated in Fig. 2, the components (10) of the air conditioning device (1) each include a base member (11), a corrosion resistant film (12) formed on the base member (11), and a hydrophilic film (13) formed on this corrosion resistant film (12). The base member (11) is formed of, for example, metal or resin. The corrosion resistant film (12) and the hydrophilic film (13) may be formed of, for example, different types of resins. The hydrophilic film (13) constitutes a film.

### Drug-containing capsule

As illustrated in Fig. 2, the hydrophilic film (13) includes therein a plurality of types of drug-containing capsules (20, 30, 40). In this example, the hydrophilic film (13) includes three types of drug-containing capsules (20, 30, 40), specifically a first drug-containing capsule (20), a second drug-containing capsule (30), and a third drug-containing capsule (40).

As illustrated in Fig. 3, the first drug-containing capsule (20) includes a plurality of (in this example, two) capsule materials (21) disposed as layers and having the shapes of hollow spheres and drugs (22) disposed within the capsule materials (21). The capsule materials (21) include degradable parts (21a) formed of a raw material that is caused to biodegrade by a specific microorganism (specifically, a polymer including caprolactone or a polymer including ethylene adipate). In this example, the capsule materials (21) are wholly constituted by the degradable parts (21a). The drugs (22) have a sterilization action for a specific microorganism and include, for example, a fungicide-bactericide and a plant essential oil. Incidentally, in the drawings, the fungicide-bactericide is illustrated in a white solid color, and the plant essential oil is illustrated as densely hatched areas.

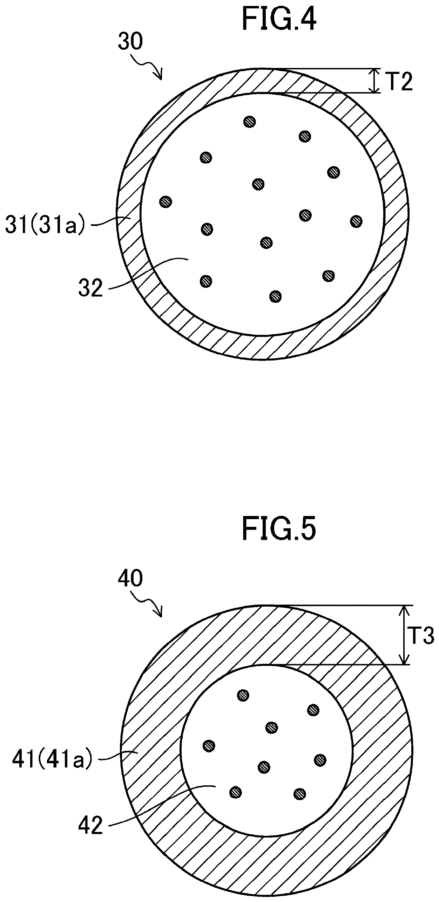

As illustrated in Fig. 4, the second drug-containing capsule (30) includes a single capsule material (31) having the shape of, for example, a hollow sphere and a drug (32) disposed within the capsule material (31). The capsule material (31) includes a degradable part (31a) formed of a raw material that is caused to biodegrade by a specific microorganism (specifically, a polymer including caprolactone or a polymer including ethylene adipate). In this example, the capsule material (31) is wholly constituted by the degradable part (31a). The drug (32) has a sterilization action for a specific microorganism and includes, for example, a fungicide-bactericide and a plant essential oil.

As illustrated in Fig. 5, the third drug-containing capsule (40) includes a single capsule material (41) having the shape of, for example, a hollow sphere and a drug (42) disposed within the capsule material (41). The capsule material (41) includes a degradable part (41a) formed of a raw material that is caused to biodegrade by a specific microorganism (specifically, a polymer including caprolactone or a polymer including ethylene adipate). In this example, the capsule material (41) is wholly constituted by the degradable part (41a). The thickness (T3) of the capsule material (41) of the third drug-containing capsule (40) is larger than the thickness (T1) of the outer capsule material (21) of the first drug-containing capsule (20) and the thickness (T2) of the capsule material (31) of the second drug-containing capsule (30) (T1 < T3, T2 < T3). The drug (42) has a sterilization action for a specific microorganism and includes, for example, a fungicide-bactericide and a plant essential oil.

Incidentally, in this Description, the "specific microorganism" means a microorganism to be destroyed by the drug-containing capsules (20, 30, 40). The microorganism is specifically a mold, a yeast, or a bacterium proliferating in the air treatment device (1): the mold includes at least one of Penicillium molds, Cladosporium molds, Aspergillus molds, Toxicocladosporium molds, Engyodontium molds, Aureobasidium molds, Alternaria molds, Paecilomyces molds, Tricoderma molds, Nigrospora molds, Chaetomium molds, Ulocladium molds, Fusarium molds, Eurotium molds, Curvularia molds, Rhodotorula molds, Chaetomium molds, Mucor molds, and Rhizopus molds; the yeast includes at least one of true fungi that are collectively called yeast; and the bacterium includes at least one of Arthrobacter bacteria, Baccilus bacteria, Serratia bacteria, and Staphylococcus aureus.

### Exertion of drug action

Hereinafter, how the drug-containing capsules (20, 30, 40) exert the drug action will be described.

For example, on the surface of the heat exchanger (10a) of the air conditioning device (1), moisture in the air condenses during a cooling operation to generate drain water, which leads to proliferation of a specific microorganism.

The proliferating specific microorganism causes biodegradation of the capsule materials (21, 31, 41) of the first to third drug-containing capsules (20, 30, 40). In this case, first, the outer capsule material (21) of the first drug-containing capsule (20) and the capsule material (31) of the second drug-containing capsule (30) are perforated. This causes release of the drugs (22, 32) from within the capsule materials (21, 31) to destroy the specific microorganism.

When the specific microorganism further proliferates, the inner capsule material (21) of the first drug-containing capsule (20) is then perforated. This causes release of the drug (22) from within the capsule material (21) to destroy the specific microorganism.

When the specific microorganism further proliferates, the capsule material (41) of the third drug-containing capsule (40) is then perforated. This causes release of the drug (42) from within the capsule material (41) to destroy the specific microorganism.

In this way, the first to third drug-containing capsules (20, 30, 40) exert the drug action at the spot and timing of the proliferation of the specific microorganism. The capsule materials (21, 31, 41) are perforated at timings different from each other, so that the drug action is exerted for a relatively long term.

### Advantages of embodiments

The drug-containing capsules (20, 30, 40) according to these embodiments include capsule materials (21, 31, 41) and drugs (22, 32, 42) disposed within the capsule materials (21, 31, 41) and having a sterilization action for a specific microorganism, wherein the capsule materials (21, 31, 41) include degradable parts (21a, 31a, 41a) formed of a raw material that is caused to biodegrade by the specific microorganism.

The inventors of the present application focused on disposition of drug-containing capsules (20, 30, 40) for a specific microorganism at the spot of proliferation of the specific microorganism. They have found that, by using a raw material that is caused to biodegrade by the specific microorganism to form at least portions of the capsule materials (21, 31, 41), the specific microorganism is destroyed at the spot and timing of proliferation. The degradable parts (21a, 31a, 41a) of the capsule materials (21, 31, 41) of the embodiments are caused to biodegrade by a specific microorganism at the spot of proliferation of the microorganism. This causes release of the drugs (22, 32, 42) from within the capsule materials (21, 31, 41) to destroy the proliferating microorganism. In this way, the drug action of the drugs (22, 32, 42) is exerted at the spot and timing of proliferation of the specific microorganism.

The specific microorganism is a mold, a yeast, or a bacterium proliferating in the air treatment device (1). Therefore, the embodiments provide drug-containing capsules (20, 30, 40) that effectively act against the mold, the yeast, or the bacterium proliferating in the air treatment device (1).

The mold includes at least one of Penicillium molds, Cladosporium molds, Aspergillus molds, Toxicocladosporium molds, Engyodontium molds, Aureobasidium molds, Alternaria molds, Paecilomyces molds, Tricoderma molds, Nigrospora molds, Chaetomium molds, Ulocladium molds, Fusarium molds, Eurotium molds, Curvularia molds, Rhodotorula molds, Chaetomium molds, Mucor molds, and Rhizopus molds; the yeast includes at least one of true fungi that are collectively called yeast; and the bacterium includes at least one of Arthrobacter bacteria, Baccilus bacteria, Serratia bacteria, and Staphylococcus aureus. The inventors of the present application have newly found that such molds, yeasts, or bacteria proliferate in the air treatment device (1). In the embodiments, a raw material that is caused to biodegrade by such a mold, a yeast, or a bacterium is used to form capsule materials (21, 31, 41). Therefore, the embodiments provide drug-containing capsules (20, 30, 40) that effectively act against such a specific mold, yeast, or bacterium.

In the drug-containing capsules (20, 30, 40) according to the embodiments, the raw material that is caused to biodegrade by such a specific microorganism is a polymer including caprolactone or a polymer including ethylene adipate. Therefore, the embodiments provide drug-containing capsules (20, 30, 40) that effectively act against the microorganism that causes biodegradation of such a polymer.

In the first drug-containing capsule (20) according to the embodiment, the capsule material (21) includes two capsule materials (21) disposed as layers, and the drug (22) is disposed within each of the capsule materials (21). Thus, upon biodegradation of the outer capsule material (21), the drug (22) disposed therein is released to destroy the specific microorganism. Subsequently, upon biodegradation of the inner capsule material (21), the drug (22) disposed therein is released to destroy the specific microorganism. In this way, such first drug-containing capsules (20) each provide a drug action twice.

The component (10) for the air treatment device (1) according to the embodiment includes the film (13) including the drug-containing capsules (20, 30, 40). Thus, the embodiment, in the air treatment device (1), effectively suppresses proliferation of the specific microorganism on the component (10) including the film (13) including the drug-containing capsules (20, 30, 40).

In the component (10) for the air treatment device (1) according to the embodiment, the film (13) includes the first to third drug-containing capsules (20, 30, 40), and, in the third drug-containing capsule (40), the thickness (T3) of the degradable part (41a) of the capsule material (41) is larger than the thicknesses (T1, T2) of the degradable parts (21a, 31a) of the capsule materials (21, 31) of the first and second drug-containing capsules (20, 30). Thus, compared with the first drug-containing capsule (20) and the second drug-containing capsule (30), in the third drug-containing capsule (40), perforation of the capsule material (21, 31, 41) due to biodegradation occurs at a later timing, so that the drug action is exerted in a later period. Therefore, compared with a case where the thicknesses (T1 to T3) of the capsule materials (21, 31, 41) of all the drug-containing capsules (20, 30, 40) are substantially the same, the total period of exerting the drug action by the first to third drug-containing capsules (20, 30, 40) becomes longer.

The drug-containing capsule (20, 30, 40) according to the embodiment includes the capsule material (21, 31, 41) including the degradable part (21a, 31a, 41a) formed of a polymer including caprolactone or a polymer including ethylene adipate, and the drug (22, 32, 42) disposed within the capsule material (21, 31, 41) and having a sterilization action for a microorganism. Thus, the degradable part (21a, 31a, 41a) of the capsule material (21, 31, 41) is caused to biodegrade by a microorganism proliferating in the air treatment device (1). This causes release of the drug (22, 32, 42) from within the capsule material (21, 31, 41) to destroy the microorganism. In this way, in the air treatment device (1), the drug action of the drugs (22, 32, 42) is exerted at the spot and timing of proliferation of the microorganism.

### <<Other embodiments>>

The above-described embodiments may be provided so as to have the following configurations.

In the above-described embodiment, the air treatment device (1) is constituted by the air conditioning device (1). Alternatively, the air treatment device (1) may be constituted by, for example, an air purification device, a humidity control device, or a ventilation device. In this case, the component (10) may be constituted by, for example, a fan or filter of the air purification device, a humidifying unit, fan, or filter of the humidity control device, or a fan, filter, or duct of the ventilation device.

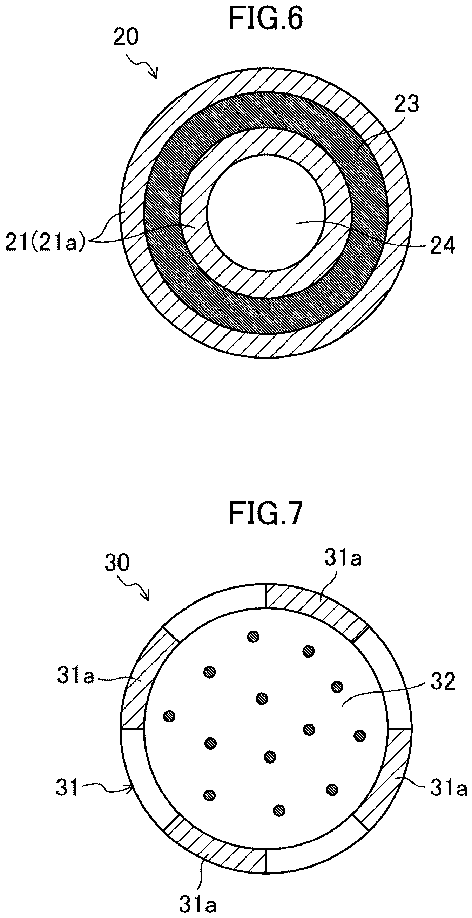

In the above-described embodiment, the drugs (22) disposed within the two capsule materials (21) of the first drug-containing capsule (20) are of the same type. Alternatively, as illustrated in Fig. 6, the type of a drug (23) disposed within the outer capsule material (21) and the type of a drug (24) disposed within the inner capsule material (21) may be different from each other. Incidentally, in this example, the drug (23) disposed within the outer capsule material (21) is a fungicide-bactericide, while the drug (24) disposed within the inner capsule material (21) is a plant essential oil. The number of the capsule materials (21) of the first drug-containing capsule (20) may be three or more.

In the above-described embodiment, the capsule materials (21, 31, 41) of the first to third drug-containing capsules (20, 30, 40) are wholly constituted by the degradable parts (21a, 31a, 41a). Alternatively, as illustrated in Fig. 7, for example, in the second drug-containing capsule (30), only portions of the capsule material (31) may be constituted by the degradable parts (31a). In this case, the degradable parts (31a) are caused to biodegrade by a specific microorganism to release the drug (32). The same applies to the first and third drug-containing capsules (20, 40).

The drug-containing capsules (20, 30, 40) included in the hydrophilic film (13) may be of two or less types, or four or more types.

The embodiments and modifications have been described so far, and it would be understood that the forms and details can be changed in various ways without departing from the spirit and scope of the Claims. The above-described embodiments and modifications can be appropriately combined or substituted unless the function of the subject matter of the present disclosure is degraded.

### Industrial Applicability

As has been described so far, the present disclosure is useful for drug-containing capsules and components for air treatment devices.

### Reference Signs List

1 air conditioning device (air treatment device)
10 component
10a heat exchanger (component)
10b fan (component)
10c drain pan (component)
10d filter (component)
13 hydrophilic film (film)
20 first drug-containing capsule (drug-containing capsule)
21 capsule material
21a degradable part
22 drug
30 second drug-containing capsule (drug-containing capsule)
31 capsule material
31a degradable part
32 drug
40 third drug-containing capsule (drug-containing capsule)
41 capsule material
41a degradable part
42 drug
T1 to T3 thicknesses (of degradable parts)

## Claims

1. A drug-containing capsule comprising:
a capsule material (21, 31, 41); and
a drug (22, 32, 42) disposed within the capsule material (21, 31, 41) and having a sterilization action for a specific microorganism,
wherein the capsule material (21, 31, 41) includes a degradable part (21a, 31a, 41a) formed of a raw material that is caused to biodegrade by the specific microorganism.

2. The drug-containing capsule according to Claim 1, wherein the specific microorganism is a mold, a yeast, or a bacterium proliferating in an air treatment device (1).

3. The drug-containing capsule according to Claim 2,
wherein the mold includes at least one of Penicillium molds, Cladosporium molds, Aspergillus molds, Toxicocladosporium molds, Engyodontium molds, Aureobasidium molds, Alternaria molds, Paecilomyces molds, Tricoderma molds, Nigrospora molds, Chaetomium molds, Ulocladium molds, Fusarium molds, Eurotium molds, Curvularia molds, Rhodotorula molds, Chaetomium molds, Mucor molds, and Rhizopus molds,
the yeast includes at least one of true fungi that are collectively called yeast, and
the bacterium includes at least one of Arthrobacter bacteria, Baccilus bacteria, Serratia bacteria, and Staphylococcus aureus.

4. The drug-containing capsule according to any one of Claims 1 to 3,
wherein the raw material that is caused to biodegrade by the specific microorganism is a polymer including caprolactone or a polymer including ethylene adipate.

5. The drug-containing capsule according to any one of Claims 1 to 4,
wherein the capsule material (21) includes a plurality of the capsule materials (21) disposed as layers, and
the drug (22) is disposed within each of the capsule materials (21).

6. A component for an air treatment device, comprising:
a film (13) including the drug-containing capsule (20, 30, 40) according to any one of Claims 1 to 5.

7. The drug-containing capsule according to Claim 6,
wherein the film (13) includes a plurality of types of the drug-containing capsules (20, 30, 40), and
the plurality of types of the drug-containing capsules (20, 30, 40) are different from each other in a thickness (T1, T2, T3) of the degradable part (21a, 31a, 41a) of the capsule material (21, 31, 41).

8. A drug-containing capsule (20, 30, 40) used for an air treatment device (1), comprising:
a capsule material (21, 31, 41) including a degradable part (21a, 31a, 41a) formed of a polymer including caprolactone or a polymer including ethylene adipate; and
a drug (22, 32, 42) disposed within the capsule material (21, 31, 41) and having a sterilization action for a microorganism.
